# EUROPEAN PATENT APPLICATION

(11) **EP 2 600 135 A1**
(43) Date of publication of application: **05.06.2013**
(21) Application number: 11812617.6
(22) Date of filing: 29.07.2011
(51) Int. Cl.: G01N 1/28, C12Q 1/34, C12Q 1/37, G01N 27/62, G01N 37/00, G01N 30/04, G01N 30/88, G01N 33/48

(54) **METHOD FOR PREPARING SUGAR CHAINS FROM ANTIBODIES**

(30) Priority: 29.07.2010 JP 2010170168
(71) Applicant: Sumitomo Bakelite Co., Ltd., Shinagawa-ku Tokyo 140-0002 (JP)
(72) Inventor: SHIMAOKA Hideyuki, Tokyo 140-0002 (JP); ABE Midori, Tokyo 140-0002 (JP)
(74) Representative: Vossius & Partner
(86) International application number: PCT/JP2011/067432
(87) International publication number: WO 2012/015028

(57) **Abstract**

A method allowing easy and quick preparation of sugar chains of antibodies is provided. Sugar chains are cut off from the antibodies in a state where the antibodies are kept being bonded to an absorbing materials, such as the Protein A or the Protein G, Then, the cut off sugar chains are captured to sugar-chain-capturing solid-phase carriers having a functional group that forms a stable bonding with the sugar chains by reacting specifically with the aldehyde groups of the sugar chains. Then, the captured sugar chains are re-released. Sugar chain samples suitable for mass spectrometry, chromatography, or electrophoresis can be prepared easily and quickly by the present method.

## Description

### TECHNICAL FIELD

The present invention relates to a method of preparing sugar chains of antibodies, and a kit and a device used for performing the method.
Priority is claimed on Japanese Patent Application No. 2010-170168, filed July 29, 2010, the content of which is incorporated herein by reference.

### BACKGROUND ART

The sugar chain is a collective term meaning a molecule, within which monosaccharides, such as glucose, galactose, mannose, fucose, xylose, N-acetylglucosamine, N-acetylgalactosamine, and sialic acid, and their derivatives being connected each other in a chain-structure through the glycoside bond.

Variety of sugar chains are known, and they are related to many functions possessed by naturally occurring organisms. The sugar chains tend to exist as a glycoconjugate, in which they are bonded to proteins or lipids, in organisms, and are important constituents in living organism. It has been known that the sugar chains in living organisms involve cellular signal transduction, regulation of protein functions and protein interaction, and the like.

In terms of the antibodies, which are the proteins responsible for immunological functions in organisms, the importance of the sugar chains, which are bonded to the antibodies recently, to the function of the antibodies has been revealed. For example, it was revealed that the structure of the sugar chains relates to the effector functions, such as the complement-dependent cytotoxicity activity (CDC activity), the antibody-dependent cellular cytotoxicity activity (ADCC activity), their half lives in blood stream, and specific diseases. Therefore, several methods of preparing sugar chains from antibodies were developed for their structural studies.

In general, the preparation of the sugar chains from antibodies divided into 4 steps. In the first step, the antibodies are purified. In the second step, the sugar chains are cut off from the antibodies. In the third step, the cut off sugar chains are labeled. In the fourth step, the labeled sugar chains are purified. Generally, affinity chromatography utilizing the Protein A, the Protein G, or the like, is adopted in the first step, where the antibodies are purified. In this method, the binding between the antibodies and the Protein A or the Protein G is performed in a column normally. Then, purified antibodies are recovered by separating the binding after washing out impurities. Therefore, this method has shortcomings of the operation being cumbersome and taking long time.

In order to solve the problem, methods with a simplified operation were developed. In these methods, the antibodies are bounded to absorbing materials, such as the Protein A, the Protein G, or the like, in the antibody purifying process. Then, the sugar chains are cut off from the antibodies in a state where the antibodies are kept being bonded with the absorbing materials (see Patent Literatures 1 and 2).
However, the method still have a shortcoming of the operations needed for purifying and labeling the cut off sugar chains from the antibodies being cumbersome.

### [Related Art Document]

### [Patent Document]

Patent Document 1: Japanese Unexamined Patent Application, First Publication No. H8-228795
Patent Document 2: PCT International Publication No. WO2009-027041

### DISCLOSURE OF INVENTION

### [Problems to be Solved by the Invention]

The present invention is made under circumstance describe above. The purpose of the present invention is to provide a method allowing easy preparation of sugar chains of antibodies in a short period of time.

### [Means for Solving the Problems]

After an intense investigation to solve the above-described shortcomings, the inventors of the present invention found followings. Sugar chain samples suitable for mass spectrometry, chromatography, or electrophoresis can be prepared easily and quickly by cutting off the sugar chains from the antibodies in a state where the antibodies are kept being bonded to an absorbing materials, such as the Protein A or the Protein G, then capturing the sugar chains to sugar-chain-capturing carriers having functional groups that form stable bondings with the sugar chains by reacting specifically with the aldehyde groups of the sugar chains, and re-releasing the sugar chains. Based on the finding, the inventors of the present invention completed the present invention.

The first aspect of the present invention is a method of preparing sugar chains of antibodies including the steps of: capturing of the antibodies, in which the antibodies are captured on carriers for capturing the antibodies by contacting a sample that contains the antibodies bonded to sugar chains with the carriers for capturing the antibodies; releasing of the sugar chains, in which the sugar chains are released from the antibodies in a state where the antibodies are kept being captured on the carriers; capturing of the sugar chains, in which the released sugar chains are captured on solid-phase carriers; and re-releasing of the sugar chains, in which the captured sugar chains are re-released from the solid-phase carriers.

In the method of preparing sugar chain of the first aspect, one or more selected from the group consisting of the Protein A, the Protein G, the Protein L, an ion-exchanging resin, and an aptamer for purifying an antibody maybe immobilized on the carriers for capturing the antibodies.

The second aspect of the present invention is a kit for performing the method of preparing sugar chains of antibodies of the first aspect of the present invention including any one of: carriers for capturing the antibodies; carriers for capturing the sugar chains; reagents for labeling the captured sugar chains and re-releasing the captured sugar chains; carriers for removing excessive reagents for labeling and re-releasing the captured sugar chains in a reaction mixture; and reaction tubes.

The third aspect of the present invention is a microfluidic passage device for performing the method of preparing sugar chains of antibodies according to the method of preparing sugar chains of antibodies of the first aspect of the present invention including: carriers for capturing the antibodies; carriers for capturing the sugar chains; and a flow passage communicating the carriers for capturing the antibodies and the carriers for capturing the sugar chains.

### [Effects of the Invention]

By the present invention, sugar chains of antibodies can be prepared easily and quickly from the antibodies contained in biological samples, making them to be applicable to the downstream analysis. Furthermore, it is possible to perform the preparation of the sugar chains in a single tube or in a single micro flow passage by the present invention, making the sugar chain samples applicable to an automated system.

### BRIEF DESCRIPTION OF THE DRAWINGS

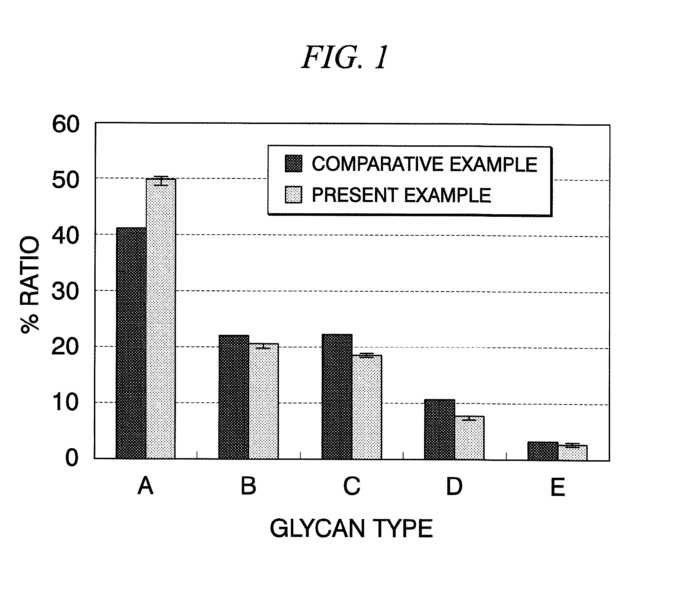

FIG. 1 is a graph comparing data of the top 5 peaks with largest area among peaks detected in an HPLC chart obtained by performing HPLC analysis of sugar chains of antibodies. The sugar chain samples are purified by the example methods of the present invention and the comparative example methods.

### BEST MODE FOR CARRYING OUT THE INVENTION

### [Step of capturing antibodies on carriers]

Examples of samples containing antibodies bonded to sugar chains used in the method of preparing sugar chain, which is the first aspect of the present invention, are body fluids, such as serum (blood), lymph fluid, peritoneal exudate, intercellular fluid, or cerebrospinal fluid, and culture supernatants of antibody-producing cells, such as B cells, hybridomas, or CHO cells. However, the present invention is not particularly limited to the examples. The carriers for capturing the antibodies in the samples are carriers on which molecules or ion-exchanging resins with affinity to antibodies are immobilized. As the molecules with affinity with antibodies, Protein A, Protein G, Protein L, an aptamer for purification of antibodies, and the like can be used for example.
The carries for capturing the antibodies are encapsulated in a container, such as a column, a multi-well titer plate, a filter plate, a micro tube, or the like. The antibodies can be captured by contacting the sample solution containing the antibodies with the carriers for capturing the antibodies.

### [Step of releasing sugar chains from the antibodies in a state where the antibodies are kept being captured]

In the sugar chain preparing method of the first aspect of the present invention, sugar chains are released from the antibodies in a state where the antibodies are kept being captured to the carriers. The operation of this method is simplified compared with the conventional method in which the sugar chains are released after the captured antibodies being eluted, recovered, desalted, and concentrated (see the method of the comparative example, which is explained later). As a method to release the sugar chains, a glycosidase treatment using N-glycosidase or O-glycosidase, hydrazine degradation, and β elimination by an alkaline treatment can be used. In a case where N-type sugar chain analysis is performed, it is preferable to adopt the method using N-glycosidase. Before the glycosidase treatment, a protease treatment, in which the trypsin and chymotrypsin proteases are used, and/or a denaturing treatment, in which a surfactant such as SDS is used, maybe performed.

### [Step of capturing the released sugar chains to solid-phase carriers]

The released sugar chains are captured on capturing carriers by contacting the solution containing sugar chains with capturing carriers that bind specifically with the sugar chains.
Sugar chains are the only substances that retain aldehyde group among the biological materials. More specifically, there is an equilibrium between the cyclic hemiacetal type and the non-cyclic aldehyde type in sugar chains. There is no aldehyde group in biological materials such as proteins, nucleic acids, and lipids. Therefore, it is possible to capture the sugar chains alone specifically by using capturing carriers having a functional group forming a stable bond reacting with the aldehyde group specifically. As a preferable functional group reacting with aldehyde group specifically, an oxyl-amino group, a hydrazide group, an amino group, a semi thio carbazide group, and their derivatives can be used, for example. More preferably, the hydrazide and oxyl amino groups can be used. A oxime bonding can be formed by reacting the oxyl amino group and the aldehyde group. A hydrazone bonding can be formed by reacting the hydrazide group and the aldehyde group. The oxime bonding and the hydrazone bonding can be broken easily by an acid treatment. Therefore, sugar chains can be easily cut off from the carriers after capturing the sugar chains. In general, the amino groups are used frequently for capturing and retention of the biologically active agents. However, the bond force of the bond formed by reacting between amino group and aldehyde group (Schiff's base) is weak. As a result, a secondary treatment using a reducing agent is needed. For this reason, the amino group is not preferable for capturing sugar chains.

It is preferable to use polymer particles as the carriers for capturing the sugar chains. It is preferable that the polymer particles are solid or gel particles which have a functional group, which reacts specifically with the aldehyde group of sugar chains, at least on a part of their surfaces. If the polymer particles are solid particles or gel particles, the polymer particles after capturing the sugar chains can be easily recovered easily by method such as centrifugation, filtration, or the like. Alternatively, a column can be filled with the polymer particles. The method using the column filled with the polymer particle particularly important to convert the method to a continuous operation. As a reaction container, a filter plate (for example, MULTISCREEN SOLVINERT FILTER PLATE manufactured by MILIPORE) can be used. By using the filter plate, multiple samples can be processed simultaneously. Thus, the throughput of sugar chain purification can be improved significantly compared with the conventional purification procedure by a column operation represented by a gel filtration.

Shapes of each grain of the polymer particles are not particularly limited. However, it is preferable that the shapes are spherical or substantially spherical. When the shape is spherical, preferable the average diameter of the grains is 0.05 µm to 1000 µm. More preferably, the average diameter of the grains is 0.1 µm to 200 µm. Even more preferably, it is 0.1 µm to 100 µm. If the average diameter of the grains is less than the lower limits, liquid permeability during filling the columns with the polymer particle is deteriorated, necessitating higher pressure to be applied. Additionally, it make it difficult to recover the particles by centrifugation or filtration. If the average diameter of the grains exceeds the upper limits, the contacting area between the polymer particles and sample solution is reduced, lowering the sugar chain capturing efficiency. In the embodiment of the first aspect of the present invention, "BLOTGLYCO (tm)" (manufacture by SUMITOMO BAKELITE COMPANY LIMITED, #BS-45603), which are polymer particles containing the hydrazide groups, can be used suitably.

Preferable pH of the reaction system during capturing the sugar chains specifically with the polymer particles is 2 to 9. More preferably, it is 2 to 7. Even more preferably, it is 2 to 6. Various buffers can be used for adjusting pH. Preferable temperature during capturing the sugar chains is 4 to 90°C. More preferably, it is 4 to 70°C. Even more preferably, it is 30 to 80°C. The most preferable temperature is a40 to 80°C. The reaction time can be set appropriately. The sample solution can be passed through a column filled with the polymer particles.

When the polymer particles are used, it is needed to wash the polymer particles since impurities other than sugar chains are absorbed non-specifically on the surface of carriers in order to remove them. As a washing solution, water, a buffer, water or buffer including a surfactant, an organic solvent, and a combination of them can be used appropriately. In the configuration particularly preferable, first the carriers are washed sufficiently with water or a buffer containing a surfactant, second washed with an organic solvent, and finally washed with water. With these washing processes, the non-specifically absorbed impurities are removed from the surfaces of the polymer particles.

### [Step of re-releasing the captured sugar chains]

Next, purified sugar chain samples are obtained by re-releasing the sugar chains binding to the polymer particles which are the capturing carriers.

The process, in which the polymer particles binding to the sugar chains are replaced with other compound (hereinafter, referred as "compound A"), is explained below. It is preferable that the compound A is a labeling reagent. The replacement occurs by adding excess amount of the compound A with respect to the amount of the polymer particles bonded with the sugar chains. Specifically, the sugar chains are freed from the polymer particles, and the compound As bind to the sugar chains at the same time (the freed sugar chains are "labeled"). A preferable amount of the excessively added compound A is 1.5-fold or more relative to the amount of the functional groups specifically react with the sugar chains on the polymer particles (as a molar ratio). More preferably, it is 3-fold or more. Even more preferably, it is 5-fold or more. The most preferable amount of the excessively added compound A is 10-fold or more. A preferable pH in the reaction system is 2 to 9. More preferably, it is 2 to 7. Even more preferably, it is 2 to 6. Various buffers can be used for adjusting pH. Preferable temperature during capturing the sugar chains is 40 to 90°C. More preferably, it is 40 to 80°C. It is preferable that the solvent is evaporated out after this process.

Preferably, the compound A is a compound having aminooxy group or hydrazide group. The most preferable compound as the compound A is N-acetyl aminooxy-tryptophanyl (arginine methyl ester). This compound is commercially available as a supplemental re-releasing agent of the previously described "BLOTGLYCO (tm) FOR MALDI" (manufacture by SUMITOMO BAKELITE COMPANY LIMITED, #BS-45603).

The method, in which the labeled samples by releasing the sugar chains captured to the sugar chain capturing substance, is explained above. However, the sugar chains are obtained as an un-labeled samples in the method described below. The first aspect of the present invention also includes such a method. In this sample preparing method, the polymer particles bonded with the sugar chains are treated under an acidic condition. By the treatment, the sugar chains are released by separating the hydrazone bonding. In a preferable condition for the acid treatment, 0.01 to 20 volume % acetic acid solution is used. Preferably, the concentration of the acetic acid solution is 0.1 to 5 volume %. In the preferable condition, reaction is performed at 40 to 80°C for 5 to 60 minutes. It is preferable that the solvent is evaporated off after this process.

In a case where the non-labeled samples are labeled with amino compounds, the method explained below can be adopted. A preferable labeling method is a reaction in which the non-labeled samples are labeled with any amino compound using the reductive amination reaction. As an amino compound, 2-aminobenzamide, 2-aminopyridine, 8-aminopyrene-1,3,6-trisulfonate, and 2-aminobenzoic acid can be used. However, the first aspect of the present invention is not particularly limited by the above-mentioned compounds. A preferable pH of the reaction system is a condition that is acidic or neutral. Preferably, it is 2 to 9. More preferably, it is 2 to 7. In terms of the temperature, preferably, it is 4 to 90°C. More preferably, it is 25 to 90°C. Even more preferably, it is 40 to 90°C. A preferable concentration of the amino compound is 1 mM to 10 M. A preferable concentration of the reducing agent is 1 mM to 10 M. Reaction time is 10 minutes to 24 hours. Preferably, the reaction time is 10 minutes to 8 hours. Even more preferably, it is 10 minutes to 3 hours. In case where the amino compound is 2-aminobenzamide particularly, a preferable pH condition is acidic or neutral. Preferably, pH is 2 to 9. More preferably, it is 2 to 8. Even more preferably, it is 2 to 7. In terms of the reaction temperature, it is 4 to 90°C. Preferably, it is 30 to 90°C. Even more preferably, it is 40 to 80°C. The concentration of the amino compound is 1 mM to 10 M. Preferably, it is 10 mM to 10 M. Even more preferably, it is 100 mM to 1 M. The concentration of the reducing agent is 1 mM to 10 M. Preferably, it is 10 mM to 10 M. Even more preferably, it is 100 mM to 2 M. The reaction time is 10 minutes to 24 hours. Preferably, it is 10 minutes to 8 hours. Even more preferably, it is 1 hour to 3 hours.

In the first aspect of the present invention, purification and labeling of the cut off sugar chains from the antibodies can be performed easily utilizing carriers containing the hydrazide group or oxyl amino group.

By using the sugar chains obtained as described above, the structure and amount of the sugar chains can be analyzed by a conventional analysis such as the mass spectrometry (MALDI-TOF MS, for example), chromatography (high performance liquid chromatography, HPAE-PAD chromatography, for example), or electrophoresis (capillary electrophoresis). In these analyses, various databases (GLYCOMOD, GLYCOSUITE, SIMGLYCAN (tm), or the like, for example) can be utilized.

### [Kit]

The present invention provides a kit for performing the method of preparing sugar chains of antibodies, which is the first aspect of the present invention, as the second aspect of the present invention. The kit includes: (1) carriers for capturing antibodies (beads or the like); (2) carriers for capturing (purifying) sugar chains (beads or the like); (3) reagents for labeling and/or re-releasing the captured sugar chains to the carriers; (4) carriers for removing the reagents (3) excessively exist in the reaction solution (a column or the like); (5) a reaction tube, and (6) an instruction.

### [Microfluidic passage device]

In the method of preparing sugar chains of antibodies, which is the first aspect of the present invention, can be performed by using a microfluidic passage device. Thus, in order to conduct the method of preparing sugar chains of antibodies of the present invention, a microfluidic passage device is also provided in the present invention. The microfluidic passage device of the present invention includes: (1) carriers for capturing antibodies; (2) carriers for capturing sugar chains; and (3) a flow passage communicating the carriers for capturing the antibodies and the carriers for capturing the sugar chains.

The microfluidic passage means a groove having width of 1 mm or less and depth of 1 mm or less in the present invention. The cross-sectional shape of the groove is not particularly limited, and it can be a rectangle , a trapezoid, a triangle, a semicircle, or a combination of them. It is preferable that the bottom surface of the groove includes a surface parallel to the surface of the device's base. In addition, there is no particular limitation to the overall shape (for example, it can be in a linear-shaped, Y-shaped, or curved-shaped), and length of the microfluidic passage, allowing to be in an appropriate configuration to fit to a specific requirement. The over all shape of the device's base can be any shape, such as a plate, a cube, a cuboid, and a sphere. However, it is preferable that the over all shape of the device's base is in a planer shape. There is no particular limitation for the material of the device's base, as long as the material has impermeability and water resistance in general. Glass, plastics, metals, or the like are named as examples of such materials.

Carriers for capturing antibodies and carriers for capturing sugar chains are provided in the microfluidic passage in the microfluidic passage device, which is the third aspect of the present invention. These carriers may be immobilized on the separate regions of the base's surface forming the passage. In addition, a weir for damming may be provided to the base material in order to dam each of the carriers at separate locations of the flow passage. Also, molecules for capturing antibodies and molecules for capturing sugar chains may be immobilized directly on the separate parts of the base's surface forming the flow passage (in this case, "carriers" mean the base material of the device).

When a sample including antibodies bonded with sugar chains is allowed to be flowed from the upstream of the microfluidic passage device, the sample reaches to the location where the carriers for capturing antibodies are provided. At this location, the antibodies are captured on the carriers. Next, a reagent for releasing sugar chains from the antibodies in a state where the antibodies are kept being captured on the carriers is allowed to be flowed in the flow passage. Sugar chains that are released by the action of the reagent reach to a downstream location where the carriers for capturing sugar chains are provided, and then are captured by the carriers. Next, a reagent for labeling and/or re-releasing the sugar chains captured on the carriers is allowed to be flowed in the flow passage. Because of these, the sugar chains captured on the carriers are re-released, allowing to obtain the purified sugar chain sample. The obtained sugar chain sample can be subjected to the sugar chain analyses described above.

### [Examples]

The present invention is explained in detail below based on the following Examples. However, the present invention is not limited by the description of the following Examples.

### [Example 1]

### [Capturing antibody]

One hundred micro liter of suspension of the Protein A beads (MABSELECT XTRA, manufactured by GE healthcare Japan Co., Ltd., 17-5269-07) was aliquoted to a reaction tube (spin column) included in the sugar chain purification kit, BLOTGLYCO (tm) (manufacture by SUMITOMO BAKELITE COMPANY LIMITED, #BS-45603). Then, the beads were washed with 200 µL of pure water three times after removing the solvent by centrifugation. Then, 1.6 mL of a culture supernatant containing antibodies was applied to the tube. Then, the beads were washed with 200 µL of pure water three times after letting initially applied supernatant passing through the spin column.

### [Releasing of sugar chains in the presence of beads]

Fifty micro liter of pure water, 5 µL of 1 M ammonium bicarbonate, and 5 µL 120 mM dithiothreitol solution were added to beads of the Protein A (50 µL) in a spin column. Then, the mixture was incubated at 60°C for 30 minutes. After allowing the temperature to drop to the room temperature, 10 µL 123 mM iodoacetaminde solution was added to the tube, and letting it stand at 37°C for 1 hour. Then, the mixture was heated at 90°C for 5 minutes to deactivate trypsin. After allowing the temperature to drop to the room temperature, 10 units ofN-GLYCOSIDASE F (manufactured by Roche Applied Science, 11365193001) was added to the tube and the tube was incubated at 37°C for 2 hours. Then, N-GLYCOSIDASE F was inactivated by heating at 90°C for 5 minutes. Finally, a solution was recovered by centrifugation of the spin column after application of 50 µL of pure water.

### [Sugar chains purification and 2AB labeling]

Sugar chain purification and fluorescent labeling (2-aminobenzenamide: 2AB) were performed with the sugar chain purification kit BLOTGLYCO (tm) (manufacture by SUMITOMO BAKELITE COMPANY LIMITED, #BS-45603) using 20 µL of the above-described solution containing sugar chains. First, 5 g of beads for capturing sugar chains was aliquoted to a reaction tube. To the reaction tube, 20 µL of the sugar chain containing solution was added first and then 180 µL of 2% acetic acid/acetonitrile solution. Then, the reaction tube was incubated at 80°C for 1 hour to allow the beads to capture the sugar chains. After the reaction, the beads were washed with 200 µL of 2 M guanidine-hydrochloride solution, 200 µL of 1% triethylamine/methanol solution, and methanol, three times each. Then, 100 µL of 10% acetic anhydride/methanol solution was added to the tube. Then, the excessive hydrazide groups present on the beads were capped by incubating the tube at the room temperature for 30 minutes. Then, the beads were washed with methanol, 10 mM hydrochloride, and water (200 µL each) sequentially. After the washing, 20 µL of pure water and 180 µL of 2% acetic acid/acetonitrile solution were added to the tube and the tube was incubated at 70 °C for 90 minutes to re-release the sugar chains from the beads. Next, 30% acetic acid/dimethylsulfoxide solution was prepared. Then, both of 2AB (350 mM) and sodium cyanoborohydride (1 M) were dissolved in the 30% acetic acid/dimethylsulfoxide solution in the specified concentrations. To the above-described beads, 50 µL of the prepared solution was added, and the mixture was incubated at 60°C for 2 hours to 2AB-label the sugar chains. After the labeling reaction, the solution was recovered to a sample tube. To the sample tube, 950 µL of acetonitrile was added to dilute it. All of the diluted solution was applied to a clean up column (a spin column with a monolithic silica filter at its bottom) included in the BLOTGLYCO (tm). The 2AB-labeled sugar chains were absorbed to the monolithic silica by letting the diluted solution pass through the column. The columns was washed with 400 µL of acetonitrile three times, and acetonitrile/pure water (95:5, v/v) three times. After the washing, the 2AB-labeled sugar chains were eluted from the monolithic silica by applying 50 µL of pure water.

### [HPLC measurement]

10 µL of the above-described labeled-sugar chain solution was subjected to HPLC measurement. As an HPLC apparatus, the model "ALLIANCE" (SEPARATION MODULE 2695, MULTI λ FLUORESCENT DETECTOR 2475) manufactured by Walters Corporation was used in a condition where the excitation wavelength was set to 330 nm and the detection wavelength was set to 420 nm. As a column for the HPLC measurement, SHODEX ASAHIPAK NH2P-50 4E was used. The column temperature was set to 40°C. The solution A (2% acetic acid/acetonitrile) and the solution B (5% acetic acid, 3% triethylamine/water) were used as eluting solutions. Gradient condition was: 30% solution B (0 minute)→ 30% solution B (2 minutes)→ 95% solution B (80 minutes)→ 95% solution B (110 minutes). Flow rate was 1 mL/minute.

### [Comparative Example]

### [Antibody purification]

Suspension of Protein A beads was loaded to a disposable column (manufactured by Varian) with a volume of 1 mL. To the disposable column, 1.6 mL of a culture supernatant including antibodies was applied, and let the supernatant pass through the column. Then, the column was washed by 1000 µL of 20 mM phosphate buffer (pH7). Then, the antibodies were eluted with 1000 µL of 100 mM glycine buffer (pH 2.7). The recovered antibody solution was desalted by dialysis. Purified antibodies were obtained by freeze-drying the desalted solution.

### [Release of sugar chain]

The purified antibodies described-above was dissolved in 50 µL of pure water. Then, 5 µL of 1 M ammonium bicarbonate solution and 5 µL of 120 mM dithiothreitol solution were added to the solution. Then, the resulting mixture was incubated at 60°C for 30 minutes. After its temperature was allowed to drop to the room temperature, 10 µL of 123 mM iodoacetaminde solution was added to the mixture. Then, the mixture was allowed to stand at room temperature in shade for 1 hour. Then, 10 units of N-GLYCOSIDASE F (manufactured by Roche Applied Science, 11365193001) was added to the mixture, and the mixture was incubated at 37°C for 2 hours. After inactivating N-GLYCOSIDASE F by heating it at 90°C for 5 minutes, proteins were precipitated by adding 80 µL of ethanol. After centrifugation, the supernatant was recovered. By freeze-drying the supernatant, sugar chains were obtained.

### [2AB labeling of sugar chains]

First, 30% acetic acid/dimethylsulfoxide solution was prepared. Then, both of 2AB (350 mM) and sodium cyanoborohydride (1 M) were dissolved in the 30% acetic acid/dimethylsulfoxide solution in the specified concentrations. To the above-described sugar chains, 50 µL of the prepared solution was added. The sugar chains were 2AB-labeled by incubating the mixture at 60°C for 2 hours. After the reaction, the solution was recovered to a sample tube and 950 µL of acetonitrile was added to dilute the sample. All of the diluted solution was applied to a clean up column (a spin column with a monolithic silica filter at its bottom). The 2AB-labeled sugar chains were absorbed to the monolithic silica by letting the diluted solution pass through the column. The columns was washed with 400 µL of acetonitrile three times, and acetonitrile/pure water (95:5, v/v) three times. After the washing, the 2AB-labeled sugar chains were eluted from the monolithic silica by applying 50 µL of pure water.

### [HPLC measurement]

HPLC measurement was performed as described in the Example of the present invention. FIG. 1 is a graph comparing the Example and the Comparative Example data of the top 5 peaks with largest area among peaks detected in an HPLC chart. It was confirmed that there is no significant difference in the sugar chain analysis between the Example and the Comparative Example. Thus, it was shown that sugar chain analysis as good as in the conventional method can be done by the simplified sugar chain preparing procedure by the method of the present invention.

### INDUSTRIAL APPLICABILITY

Sugar chain samples can be prepared from antibodies contained in biological samples in a simple procedure and quickly by the method of preparing sugar chains of antibodies of the present invention. The present invention contributes studies of sugar chains of antibodies, and diagnosis of diseases related to the sugar chain structure in antibodies, significantly. The present invention contributes research/development, production, and quality control of antibody drugs too.

## Claims

1. A method of preparing sugar chains of antibodies comprising the steps of:
capturing of the antibodies, in which the antibodies are captured on carriers for capturing the antibodies by contacting a sample that contains the antibodies bonded to sugar chains with the carriers for capturing the antibodies;
releasing of the sugar chains, in which the sugar chains are released from the antibodies in a state where the antibodies are kept being captured on the carriers;
capturing of the sugar chains, in which the released sugar chains are captured on solid-phase carriers; and
re-releasing of the sugar chains, in which the captured sugar chains are re-released from the solid-phase carriers.

2. The method of preparing sugar chains of antibodies according to Claim 1, wherein one or more selected from the group consisting of the protein A, the protein G, the protein L, an ion-exchanging resin, and an aptamer for purifying an antibody are immobilized on the carriers for capturing the antibodies.

3. A kit for performing the method of preparing sugar chains of antibodies according to Claim 1 comprising any one of:
carriers for capturing the antibodies;
carriers for capturing the sugar chains;
reagents for labeling the captured sugar chains and re-releasing the captured sugar chains;
carriers for removing excessive reagents for labeling and re-releasing the captured sugar chains in a reaction mixture; and
reaction tubes.

4. A microfluidic passage device for performing the method of preparing sugar chains of antibodies according to Claim 1 comprising:
carriers for capturing the antibodies;
carriers for capturing the sugar chains; and
a flow passage communicating the carriers for capturing the antibodies and the carriers for capturing the sugar chains.
